# EUROPEAN PATENT APPLICATION

(11) **EP 2 135 515 A1**
(43) Date of publication of application: **23.12.2009**
(21) Application number: 08158659.6
(22) Date of filing: 20.06.2008
(51) Int. Cl.: A23L 1/226, C07C 43/23

(54) **Taste enhancing compound**

(71) Applicant: FIRMENICH SA, 1211 Genève 8 (CH)
(72) Inventor: Hofmann, Thomas, 85375, NEUFAHRN (DE); Schwarz, Bernd, 46514, SCHERMBECK (DE)
(74) Representative: Dale, Gavin Christopher

(57) **Abstract**

The invention relates to the use of a compound according to Formula (I) 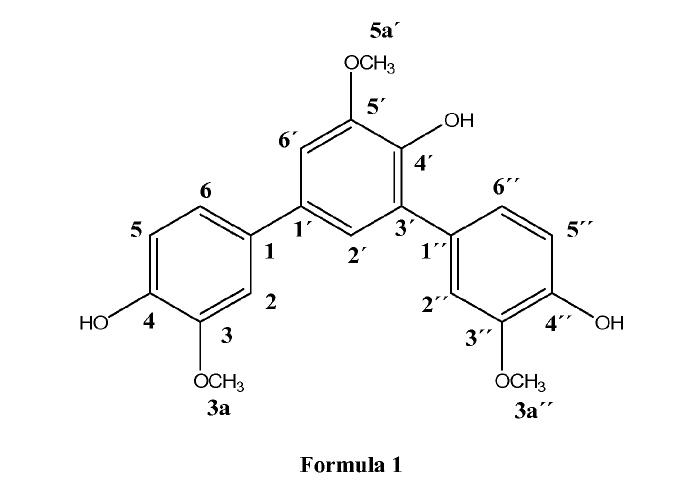 to confer, enhance, improve or modify the mouthfeel properties of a flavouring composition or of a flavoured article.

## Description

### Technical Field

The present invention relates to the field of flavour. More particularly, it concerns a compound that can be used to enhance taste, in particular to impart a mouthfeel effect.

### Background and Prior Art

Treating food products to reduce or even remove their fat or sugar content is a very common procedure in the foods industry in order to provide reduced calorie foods. However, fats and sugars are a very important component for the taste of a product and especially for the feeling in the mouth that they provide. Whilst the taste characteristic of fats and sugars can be easily replicated, there remains the problem related to the difficulty in reconstituting or reproducing the feeling in the mouth or "mouthfeel", particularly the fullness or creamy-type consistency or texture which fats and sugars give. This problem is particularly acute in fat- or sugar-reduced dairy products, carbonated beverages, margarines, mayonnaises, frozen desserts and the like.

The triguaiacol of interest in the present invention has been described in the publication Guaiacol Oxidation Products in the Enzyme-Activity Assay Reaction by Horseradish Peroxidase Catalysis, Tonami, Uyami, Nagahata and Kobayashi, Chemistry Letters Vo1.33, No. 7 (2004) p796-797, though there is no disclosure or suggestion of the taste modifying effects of this compound. Surprisingly, the present inventors have found that this compound delivers a velvety mouthfeel effect when consumed, even at extremely low dosages.

### Summary of the Invention

Accordingly, the present invention relates to the use of a compound according to formula (I): 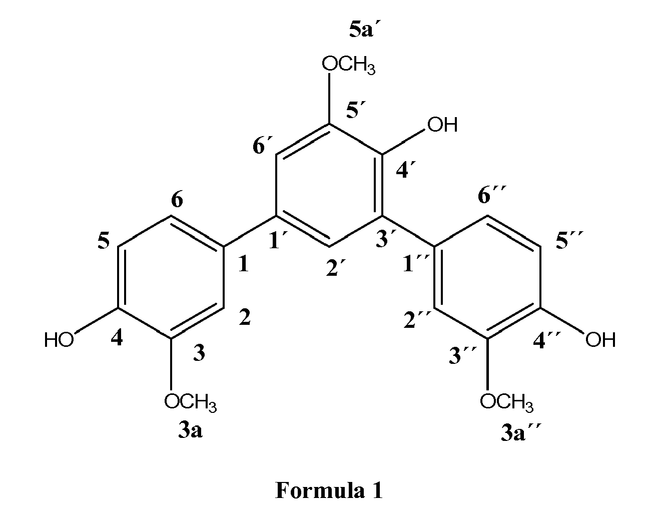 to confer, enhance, improve or modify the mouthfeel properties of a flavouring composition or of a flavoured article.

In a further aspect the invention relates to a flavouring composition or flavoured article comprising a mouthfeel-enhancing effective amount of the composition described above.

In the context of the present invention, formula (I) is not limited to the structure as shown but includes all stereo-isomers thereof.

### Detailed Description

The taste enhancing compound according to formula (I) can be prepared and isolated according to the method described in the publication Guaiacol Oxidation Products in the Enzyme-Activity Assay Reaction by Horseradish Peroxidase Catalysis, Tonami, Uyami, Nagahata and Kobayashi, Chemistry Letters Vol.33, No. 7 (2004) p796-797.

To the best of our knowledge, the compound according to formula (I) is not present in either green or cured vanilla beans, but results from the enzymatic reaction of vanillin and guaiacol as described herein.

The compound of the invention can be used alone or in the form of a flavouring composition, that is together with one or more further flavouring and taste-imparting substances.

The term "flavouring composition" has the usual meaning in the art in that it is a composition which imparts a hedonic effect, i.e. is able to impart or modify in a pleasant way the taste of a preparation, and not just as imparting a taste.

The proportions in which the compound according to the invention can be incorporated into the various compositions vary within a wide range of values. These values are dependent on the nature of the article to be flavoured and on the desired organoleptic effect as well as the nature of the co-ingredients in a given base when the compound according to the invention are mixed with flavouring co-ingredients, solvents or additives commonly used in the art. Typical examples of said proportions are given above.

By "flavour carrier", it is meant a material that is substantially neutral in terms of flavour, such that it does not significantly alter the organoleptic properties of flavouring ingredients. The carrier may be a liquid or a solid.

Suitable liquid carriers include, for example, an emulsifying system, i.e. a solvent and a surfactant system, or a solvent commonly used in flavours. Suitable solvents include, for example, propylene glycol, glycerin, triacetine, triethyl citrate, benzylic alcohol, ethanol, vegetal oils or terpenes. This list is non-exhaustive and the skilled person will readily understand that other solvents may be suitable for use in the present invention.

Suitable solid carriers include, for example, absorbing gums or polymers, or even encapsulating materials. Examples of such materials include wall-forming and plasticizing materials, such as mono, di- or trisaccharides, natural or modified starches, hydrocolloids, cellulose derivatives, polyvinyl acetates, polyvinylalcohols, proteins or pectins, or the materials cited in reference texts such as H. Scherz, Hydrokolloids : Stabilisatoren, Dickungs- und Geliermittel in Lebensmittel, Band 2 der Schriftenreihe Lebensmittelchemie, Lebensmittelqualität, Behr's Verlag GmbH & Co., Hamburg, 1996. Encapsulation is a well known process to a person skilled in the art, and may be performed, for instance, using techniques such as spray-drying, agglomeration and extrusion; or it may consist of a coating encapsulation, such as simple or complex coacervation techniques.

By "flavour base", it is meant a composition comprising at least one flavouring co-ingredient.

Said flavouring co-ingredient is not of formula (I). Moreover, by "flavouring co-ingredient" it is meant a compound, which is used in flavouring preparation or composition to impart a hedonic effect. In other words such a co-ingredient, to be considered as being a flavouring one, must be recognized by a person skilled in the art as being able to impart or modify in a positive or pleasant way the odour or taste of a composition, and not just as having a taste.

The nature and type of the flavouring co-ingredients present in the base do not warrant a more detailed description here, which in any case would not be exhaustive, the skilled person being able to select them on the basis of its general knowledge and according to intended use or application and the desired organoleptic effect. Typically, these flavouring co-ingredients belong to chemical classes as varied as alcohols, aldehydes, ketones, esters, ethers, acetates, nitriles, terpene hydrocarbons, nitrogenous or sulphurous heterocyclic compounds and essential oils. The co-ingredients can be of natural or synthetic origin. Many of these co-ingredients are in any case listed in reference texts such as the book by S. Arctander, Perfume and Flavour Chemicals, 1969, Montclair, New Jersey, USA, or its more recent versions, or in other works of a similar nature, as well as in the abundant patent literature in the field of flavour. It is also understood that said co-ingredients may also be compounds known to release in a controlled manner various types of flavouring compounds.

By "flavour adjuvant", it is meant an ingredient capable of imparting additional added benefit such as a colour, a particular light resistance, chemical stability, and so on. A detailed description of the nature and type of adjuvant commonly used in flavouring bases cannot be exhaustive, though such ingredients are well known to a person skilled in the art.

A composition consisting of the compound according to formula (I) and at least one flavour carrier represents a particular embodiment of the invention. A further embodiment is represented by a flavouring composition comprising the compound according to formula (I), at least one flavour carrier, at least one flavour base, and optionally at least one flavour adjuvant.

The potency of the compound according to formula (I) for delivering velvety mouthfeel makes it suitable for incorporation into low-fat and reduced-fat foodstuffs and beverages.

Examples of low-fat and reduced-fat products include low-fat yoghurts, diet beverages, low-fat cheeses, reduced-fat milk, instant desserts and low-fat ice-creams. Low fat yoghurts and diet beverages are particularly suitable since these are often associated with vanilla flavours. Thus, it can be envisaged that the compound according to formula (I) could be added to further enhance the velvety mouthfeel of the vanilla-flavoured article.

In the flavoured article, the compound according to formula (I) can be present at very low levels. Thus, it is preferred that the compound is present in an amount of from 1x10⁻⁴wt% to 1wt%, based on the total weight of the flavoured article.

In a flavouring composition, the compound according to formula (I) is preferably present in an amount within the range of from 0.1 to 20 % by weight, relative to the total weight of the flavouring composition. At these levels, the desired mouthfeel enhancing effect is achieved. Below this level, the mouthfeel effect is insufficiently perceived whilst, above this level, the presence of secondary, often undesirable, notes and tastes becomes more apparent.

It is to be noted that the invention thus provides flavouring compositions capable of improving or imparting the organoleptic characters generally associated with the presence of fats and sugars, namely the creamy texture and the enhanced and more voluminous mouthfeel. Therefore, the compound according to formula (I) is able to replace, totally or partially, said fat or sugar materials in a great variety of light or low-calorie foodstuffs, but it can also serve to enhance the organoleptic properties thereof in the foodstuffs which already contain said fat or sugar. The foodstuffs which comprise the flavouring compound according to formula (I) have an enhanced impact and a longer-lasting taste in the mouth, as well as better mouthfeel, than the corresponding foods not flavoured according to the invention.

### Examples

The invention will now be illustrated with reference to the following examples. All amounts are in % by weight unless otherwise indicated.

### Example 1

### Isolation of the Compound According to Formula (I)

The following reaction was performed. In a 50ml flask containing 25ml of a buffered aqueous solution (pH 4.0), 5mmol guaiacol (ex Sigma-Aldrich), 5mmol vanillin (ex Sigma-Aldrich) and 1mg horseradish peroxidase (ex Sigma-Aldrich) were introduced. Hydrogen peroxide (5% aqueous solution, 3.4ml, 5mmol) was added dropwise to the mixture for 2 hours at 40 to 50°C under air. The reaction was then left to continue for 18 hours after which time, it was stopped by addition of methanol. The resulting precipitate was collected by centrifugation and washed with water repeatedly, followed by drying in vacuo.

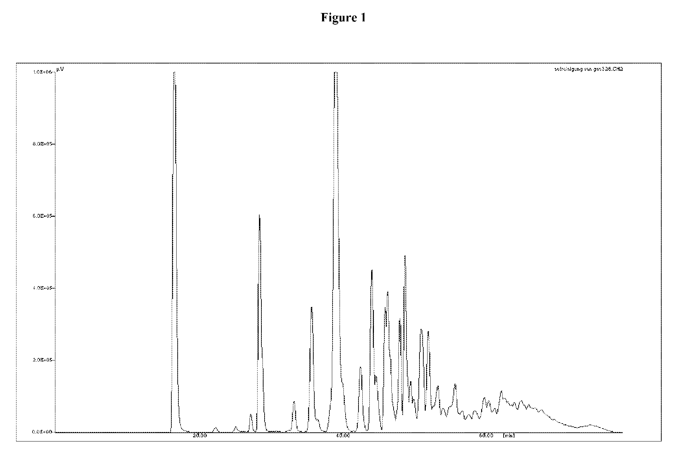

The precipitate was analysed by preparative HPLC at 272nm. The results are shown in Figure 1.

The compound identified by the absorption peak having an intensity of 1.0 E+6µV and a retention time of about 38 to 42 minutes was separated and analysed by UV/VIS, LC-MS/MS and NMR. The results are as follows:
UV/VIS (Acetonitrile): λₘₐₓ = 259 nm
LC-MS (ESI⁻): *m*/*z* 367.2 ([M-H]⁻), 294.1 ([M-H-73]⁻);
¹H NMR (400 MHz, DMSO-d₆; COSY): δ 3.72 [s, 3H, H-C(3a')], 3.77 [s, 3H, H-C(3a")], 3.84 [s, 3H, H-C(5a')], 6.736 [d, *J=* 8.1 Hz, 1H, H-C(5")], 6.743 [d, *J=* 8.1 Hz ,1H, H-C(5)], 6.94 [dd, *J=* 2.0 Hz, 8.1 Hz, 1H, H-C(6")], 6.97 [d, *J=* 2.0 Hz, 1H, H-C(6')], 6.98 [dd, *J=* 2.0, 8.1 Hz, 1H, H-C(6)], 7.02 [d, *J=* 2.0 Hz, 1H, H-C(2')], 7.07 [d, *J=* 2.0 Hz, 1H, H-C(2")], 7.08 [d, *J=* 2.0 Hz, 1H, H-C(2)], 8.40 [s, 1H, H-O(4')], 8.89 [s, 1H, H-O(3a")], 8.90 [s, 1H, H-O(3a')].
¹³C NMR (100 MHz, DMSO-d₆; HMQC, HMBC): δ 148.3 [C(3)], 148.1 [C(5')], 147.9 [C(4)], 147.3 [C(3")], 147.1 [C(4")], 142.7 [C(4')], 132.5 [C(1")], 132.3 [C(1)], 130.2 [C(1')], 128.9 [C(3')], 122.2 [C(6")], 120.3 [C(6')], 118.8 [C(6)], 116.4 [C(5)], 115.3 [C(5")], 114.4 [C(2)], 114.1 [C(2")], 108.9 [C(8)], 56.4 [C(3a)], 56.1 [C(5a', 3a")].

### Example 2

### Sensory Analysis of the Compound According to Formula (I)

The threshold at which the compound delivered a noticeable velvety mouthcoating was evaluated as follows:

The isolated compound was suspended in water, and, after removing the volatiles in high vacuum (<5 mPa), was freeze-dried twice.

The freeze-dried sample was taken up in water, adjusted to pH 4.5 using hydrochloric acid, and stepwise diluted (1+1) with water. This is referred to herein as "sample 1". A second sample was prepared comprising water (Evian®) adjusted to pH 4.5 using hydrochloric acid. This is referred to herein as "sample 2". Both samples were stored at room temperature.

Twelve panelists with no history of known taste disorders were trained to evaluate the taste of aqueous solutions (2 mL each) of the following standard taste compounds in bottled water (pH 4.5) by using a triangle test as described in the literature (20): sucrose (50 mmol/L) for sweet taste, lactic acid (20 mmol/L) for sour taste, NaCl (20 mmol/L) for salty taste, caffeine (1 mmol/L) for bitter taste, sodium glutamate (3 mmol/L) for umami taste. For the puckering astringency and the velvety astringent, mouth-drying oral sensation, the panel was trained by using gallustannic acid (0.05%) and quercetin-3-*O*-β-D-glucopyranoside (0.01 mmol/L), respectively, using the half-tongue test (see Scharbert S., Holzmann N., Hofmann T. Identification of the astringent taste compounds in black tea infusions by combining instrumental analysis and human bioresponse. J. Agric. Food Chem. 2004, 52, 3498-3508).

Samples 1 and 2 were presented in order of increasing concentrations to the panel. The panelists, wearing noseclips, were asked to determine the dilution at which a difference was detectable between the samples using the "half-tongue" test. The test was performed three times. When the panelist selected correctly, the same concentration was presented again besides one blank as a proof for the correctness of the data. The geometric mean of the last and the second last concentration was calculated and taken as the individual recognition threshold. The values between individuals and between five separate sessions differed by not more than plus or minus one dilution step.

For comparison with the mouthfeel-enhancing threshold of known ingredients in vanilla extract, the half-tongue test was repeated using vanillic acid, p-hydroxybenzoic acid, guaiacol, p-hydroxybenzaldehyde and vanillin respectively instead of sample 1.

The results are given in the following table:

**Table 1**

| Sample | Mouthfeel threshold (µmol/L) |
|---|---|
| Vanillic acid* | 320 |
| p-hydroxybenzoic acid* | 250 |
| Guaiacol* | 240 |
| p-hydroxybenzaldehyde* | 205 |
| Vanillin* | 150 |
| Sample 1 | 0.01 |

| | |
|---|---|
| * ex Aldrich | |

The results demonstrate that the compound according to the invention enhances mouthfeel at exceptionally low levels compared to known mouthfeel ingredients present in vanilla extract.

### Example 3

### Diet beverage

Two diet cola syrups were prepared by admixing the following ingredients:

**Table 2**

| Ingredient | Cola "A" (parts by weight/volume) | Cola "B" (parts by weight/volume) |
|---|---|---|
| Cola Flavour (1) | 6.0g | 6.0g |
| Caramel BS 400 | 4.44g | 4.44g |
| Aspartame | 2.22g | 2.22g |
| Acesulfame-K | 0.26g | 0.26g |
| Sodium benzoate (25 % w/v solution) | 7.0ml | 7.0ml |
| Phosphoric acid (85 % w/v solution) | 3.0ml | 3.0ml |
| Citric acid (50 % w/v solution) | 1.5ml | 1.5ml |
| Caffeine | 0.78ml | 0.78ml |
| Compound according to the invention | 0 | 0.01g |
| QS with water to | 1000ml | 1000ml |

| | | |
|---|---|---|
| (1) ex Firmenich S.A., Geneva Switzerland (ref. 599077T) | | |

The addition of 1 part by volume of composition "A" or "B" to 5 parts carbonated water provided two new diet cola beverages, respectively A' and B'.

The flavour of diet cola B' in a blind test was described as having a better mouthfeel, also described as being more velvety than diet cola A'.

### Example 4

### Low Fat Puddings

Vanilla and chocolate puddings with 0% fat were prepared in a standard manner using the ingredients given in the following table (all amounts denote parts by weight).

**Table 3**

| Ingredient | Pudding A | Pudding A' | Pudding B | Pudding B' |
|---|---|---|---|---|
| Granulated sugar | 69.10 | 69.10 | 69.10 | 69.10 |
| Dariloid® QH (1) | 3.00 | 3.00 | 3.00 | 3.00 |
| Powdered tetrasodium pyrophosphate | 1.30 | 1.30 | 1.30 | 1.30 |
| Calcium sulphate dehydrate | 0.70 | 0.70 | 0.70 | 0.70 |
| Salt | 0.40 | 0.40 | 0.40 | 0.40 |
| Food grade Colouring | 0.47 | 0.47 | 0.47 | 0.47 |
| Vanillin | 0.03 | 0.03 | 0.03 | 0.03 |
| Vanilla Flavour (2) | 0.60 | 0.60 | - | - |
| Chocolate Flavour (3) | - | - | 0.60 | 0.60 |
| Compound of the Invention | - | 0.30 | - | 0.30 |

| | | | | |
|---|---|---|---|---|
| (1) Alginate, ex Kelko International GmbH, Germany (2) Vanilla 54.286 BP0551, ex Firmenich SA, Switzerland (3) Chocolate 503.313 AP0551, ex Firmenich SA, Switzerland | | | | |

Each pudding was tasted by a panel of expert flavourists. Puddings A' and B' were found to have a longer lasting and more rounded flavour that puddings A and B respectively.

### Example 5

### Low Fat Frozen Desert

A frozen desert was prepared using the ingredients in the following table:

**Table 4**

| Ingredient | % by weight |
|---|---|
| Skimmed milk (0% fat) | 71.53 |
| Litesse® (1) | 11.00 |
| Sorbex® (2) | 6.00 |
| Powdered skimmed milk | 5.00 |
| Glucidex® 12 (3) | 4.00 |
| Paselli® SA2 (4) | 2.00 |
| Meyprogen® IC 304 (5) | 0.40 |
| Aspartame® (6) | 0.07 |

| | |
|---|---|
| (1) sugar substitute; ex Pfizer AG, Zurich, Switzerland (2) sorbitol; ex Hefti AG, Zurich, Switzerland (3) maltodextrin; ex Roquette Frères, France (4) modified starch; ex Avebe International, Holland (5) emulsifier; ex Meyhall Chemical AG, Switzerland (6) ex Nutrasweet AG, Switzerland | |

These ingredients were mixed and the dessert was frozen according to standard methods. At the same time, a dessert flavoured according to the invention was prepared by adding to the dessert above-mentioned, before freezing, 0.3% by weight of the compound of the invention described in the preceding examples.

The two frozen desserts were then evaluated on a blind test by a panel of expert flavourists, who showed unanimous preference for the dessert flavoured according to the invention. In their opinion, its taste had an enhanced impact in the mouth and it was also longer lasting with an improved mouthfeel.

## Claims

1. The use of a compound according to Formula (I) 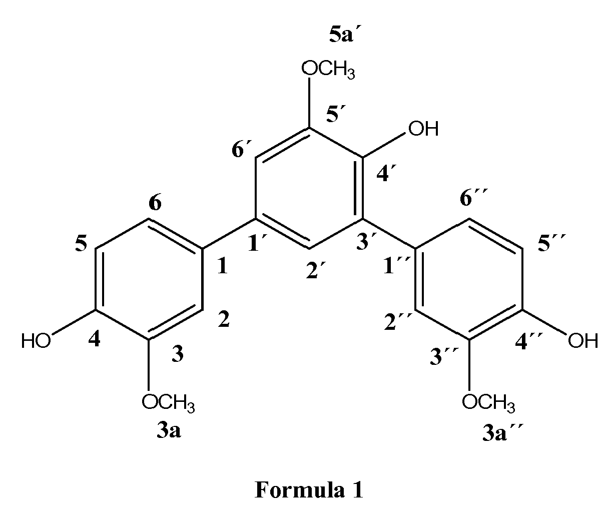 to confer, enhance, improve or modify the mouthfeel properties of a flavouring composition or of a flavoured article.

2. A flavouring composition or flavoured article comprising a mouthfeel-enhancing effective amount of the compound according to claim 1.

3. A flavoured article according to claim 2 wherein the article comprises a low fat yoghurt.

4. A flavoured article according to claim 2 wherein the article comprises a diet carbonated beverage.

5. A flavoured article according to claim 5 or claim 4 wherein the compound is present in an amount of from 1x10⁻⁴wt% to 1 wt%, based on the total weight of the flavoured article.

6. A flavouring composition according to claim 4 wherein the compound is present in an amount of from 0.1 wt% to 20wt%, based on the total weight of the flavouring composition.
